# EUROPEAN PATENT SPECIFICATION

(11) **EP 3 443 963 B1**
(45) Date of publication and mention of the grant of the patent: **25.03.2020**
(21) Application number: 17782633.6
(22) Date of filing: 11.04.2017
(51) Int. Cl.: A61K 31/52, A23L 33/10

(54) **PHARMACEUTICAL COMPOSITION FOR SUPPRESSING APPETITE, CONTAINING N2-(M-TRIFLUOROBENZYL), N6-(P-NITROBENZYL)PURINE (TNP) OR PHARMACEUTICALLY ACCEPTABLE SALT THEREOF AS ACTIVE INGREDIENT**
PHARMAZEUTISCHE ZUSAMMENSETZUNG ZUR APPETITZÜGELUNG MIT N2-(M-TRIFLUORBENZYL), (N6-P-NITROBENZYL)PURIN (TNP) ODER EINEM PHARMAZEUTISCH AKZEPTABLEN SALZ DARAUS ALS WIRKSTOFF
COMPOSITION PHARMACEUTIQUE DESTINÉE À SUPPRIMER L'APPÉTIT, CONTENANT DU N2- (M-TRIFLUOROBENZYLE), DE LA N6- (P-NITROBENZYL)PURINE (TNP) OU UN SEL PHARMACEUTIQUEMENT ACCEPTABLE DE CETTE DERNIÈRE EN TANT QUE PRINCIPE ACTIF

(30) Priority: 13.04.2016 KR 20160045222
(43) Date of publication of application: 20.02.2019
(73) Proprietor: Korea Advanced Institute of Science and Technology, Daejeon 34141 (KR); University Of Ulsan Foundation For Industry Cooperation, Ulsan 44610 (KR); The Asan Foundation, Seoul 05505 (KR)
(72) Inventor: KIM, Seyun, Seoul 06592 (KR); KIM, Min Seon, Seoul 05649 (KR); KANG, Gilmyoung, Seongnam-si Gyeonggi-do 13476 (KR)
(74) Representative: Abel & Imray
(86) International application number: PCT/KR2017/003892
(87) International publication number: WO 2017/179878

(56) References cited:
- KR-A- 20080 010 746
- KR-B1- 100 848 747
- KR-B1- 101 671 008
- SARBANI GHOSHAL ET AL: "TNP [N2-(m-Trifluorobenzyl), N6-(p-nitrobenzyl)purine] ameliorates diet induced obesity and insulin resistance via inhibition of the IP6K1 pathway", MOLECULAR METABOLISM, vol. 5, no. 10, 1 October 2016 (2016-10-01), pages 903-917, XP055539759, ISSN: 2212-8778, DOI: 10.1016/j.molmet.2016.08.008
- DANIELA COTA, KARINE PROULX, KATHI A. BLAKE SMITH, SARA C. KOZMA, GEORGE THOMAS, STEPHEN C. WOODS AND RANDY J. SEELEY: "Hypothalamic mTOR Signaling Regulates Food Intake", SCIENCE, vol. 312, no. 5775, 12 May 2006 (2006-05-12), pages 927-930, XP055539954, US ISSN: 0036-8075, DOI: 10.1126/science.1126241
- CHAKRABORTY, A. et al.: "Inositol Pyrophosphates Inhibit Akt Signaling, Thereby Regulating insulin Sensitivity and Weight Gain", Cell, vol. 143, no. 6, 2010, pages 897-910, XP055430936,
- PADMANABHAN, U. et al.: "Characterization of a Selective Inhibitor of Inositol Hexakisphosphate Kinases", The Journal of Biological Chemistry, vol. 284, no. 16, 2009, pages 10571-10582, XP055430939,
- MACKENZIE, R. et al.: "Akt/PKB Activation and Insulin Signaling: A Novel Insulin Signaling Pathway in the Treatment of Type 2 Diabetes", Diabetes, Metabolic Syndrome and Obesity: Targets and Therapy, vol. 7, 2014, pages 55-64, XP055561172,

## Description

### BACKGROUND OF THE INVENTION

### 1. Field of the Invention

The present invention relates to a pharmaceutical composition or a nutraceutical food for suppressing appetite containing TNP (N2-(m-Trifluorobenzyl), N6-(p-nitrobenzyl)purine) or a pharmaceutically acceptable salt thereof as an active ingredient.

### 2. Description of the Related Art

TNP (N2-(m-Trifluorobenzyl), N6-(p-nitrobenzyl)purine) is a commercially available chemical that is an inositol hexakisphosphate kinase specific inhibitor (Padmanabhan et al., J. Biol. Chem. 2009). According to previous reports, TNP inhibits the biosynthesis of 5-inositol pyrophosphate, thereby lowering the level of intracellular 5-inositol polyphosphate and increasing the insulin signaling response of hepatocytes (Chakraborty et al., Cell, 2010. 143(6):897-910). When TNP is treated to stem cells, it can inhibit aging of the cells (Zhang et al., Stem Cell Res Ther. 2014).

There is no effective treatment method for obesity which is defined as excessive fat in the body, yet. However, the world-wide obesity population continues to increase. The world-wide obesity incidence became almost double from 1980 to 2008. In fact, it is known that about 11% of the world's population [total: about 500 million people (male: 200 million, female: 300 million)] is obesity patients (BMI 30 kg/m² or more) and this trend is growing very rapidly. In the United States, about 20.6% of the total medical expenses, about 192 billion US dollars (about 219 trillion Korean Won), are spent for the regulation and treatment of obesity. Considering the fast increasing costs for the treatment of obesity, additional medical expenses of about 630 trillion Korean Won (555 billion US dollars) will be used for the next 20 years to regulate and treat obesity. In Korea, 3.5% of the total population (1.5 ∼ 2 million people) is obesity patients, which is also fast increasing due to westernization and diversification of dietary habits.

Many countries in the world are trying to develop an efficient obesity treatment agent to overcome the problems mentioned above. Due to the advancement of biotechnology, it is expected that the development of effective and safe therapeutic agents will expand rapidly in the future.

The risk of obesity and various complications (eg, cancer, myocardial infarction, stroke, hypertension, etc.) caused by obesity has been a world-wide issue, but there are still no efficient obesity treating drugs developed so far. Therefore, it is anticipated that the development of a noble drug for treating obesity with a novel pharmaceutical mechanism and less side effects and a global market entry will be expected.

The conventional obesity treating agents are largely divided into two groups; one is the drug to suppress the absorption of fat included in the food taken or to increase the consumption of energy and the other is an anorexing agent to suppress appetite by working on central nervous system. The former is limited in treating obesity because even though the digest of food taken in is suppressed or the generated fat is decomposed, such suppression of digest or induced lipolysis will not be efficient when food is continuously taken and accordingly energy is continuously supplied in the body. The representative anorexing agents are fullerin and pentysin comprising pentimetrazine. These drugs are fast and strong to suppress appetite and are not expensive. However, these drugs are classified as narcotics according to Ministry of Food and Drug Safety, Korea, so it is neither allowed to take these drugs more than a month nor prescribed with other drugs together. In the meantime, reductil, linoban, and slimmer comprising sibutramine as an active ingredient had been voluntarily recalled in 2010 in Korea following the US and Europe due to the high risk of cardiovascular disease.

Recently, anorexing agents that act on the central nervous system have been developed as new drugs for obesity treatment due to the research on drug development which is related to obesity (International Journal of Obesity (2013) 37, 107117; Neuropharmacology (2012) 63, 132146). As an example, FDA (USA) approved two new anorexing agents, Belviq (locarserin) and Qsymia (Phentermine + Toiramide), in
2012. Exernatide (Byetta) and Liraglutide (Victoza), the GLP-1 (Glucagon-Like peptide) peptides, have been approved and are now being sold as therapeutic agents for type 2 diabetes, which are efficient in suppressing appetite by increasing satiety. According to the evaluation made by domestic medical staffs, Belviq is a drug that can be used comfortably without side effects, but it cannot expect a remarkable weight loss effect and it is expensive. Qsymia displays better weight reducing effect than Belviq but is not yet introduced in Korea and is not recommended for those patients with cardiovascular disease since the drug is not approved yet in Europe because of the risk in cardiovascular brain disease.

It has also been reported that hypothalamic mTOR signaling (Cota et al., Science, 2006, 312, 927-930) and activation of Atk/PKB (Mackenzie et al., Metabolic Syndrone and Obesity: Target and Thrapy, 2014, 7, 55-64) may provide useful avenues for modulating insulin signaling and decreasing body weight.

Considering that the development of a novel anorexing agent for the treatment of obesity is urgent, the present inventors have tried to screen a material to inhibit weight gaining and to suppress appetite. In the course of our study, the inventors confirmed that when TNP was administered to the central nervous system of a mouse animal model, the food intake behavior was reduced in the normal diet mouse, resulting in the suppression of weight gaining. Accordingly, the present inventors confirmed that the composition comprising TNP or a pharmaceutically acceptable salt thereof of the invention can be effectively used as a pharmaceutical composition and a nutraceutical food for suppressing appetite, leading to the completion of the present invention.

### SUMMARY OF THE INVENTION

It is an object of the present invention to provide a pharmaceutical composition and a nutraceutical food for use in suppressing appetite in a subject in need thereof comprising TNP (N2-(m-Trifluorobenzyl), N6-(p-nitrobenzyl)purine) or a pharmaceutically acceptable salt thereof as an active ingredient.

To achieve the above objects, the present invention provides a pharmaceutical composition for use in suppressing appetite in a subject in need thereof comprising the TNP (N2-(m-Trifluorobenzyl), N6-(p-nitrobenzyl)purine) compound represented by formula 1 below or a pharmaceutically acceptable salt thereof as an active ingredient:

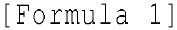

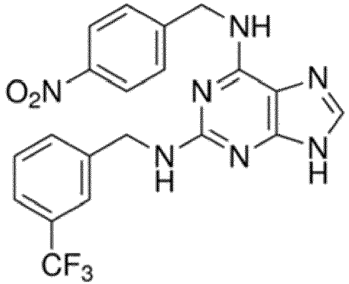

In addition, the present invention provides a nutraceutical food for suppressing appetite comprising the TNP compound represented by formula 1 above or a pharmaceutically acceptable salt thereof as an active ingredient.

### ADVANTAGEOUS EFFECT

In this invention, it was confirmed that the food intake behavior was reduced in normal mice and the weight gain was suppressed when TNP (N2-(m-Trifluorobenzyl), N6-(p-nitrobenzyl)purine) was administered to the central nervous system of a mouse animal model. Therefore, TNP or a pharmaceutically acceptable salt thereof can be effectively used for the development of a drug for suppressing appetite which is working basically on the central nervous system.

### BRIEF DESCRIPTION OF THE DRAWINGS

The application of the preferred embodiments of the present invention is best understood with reference to the accompanying drawings, wherein:
Figure 1 is a diagram illustrating the appetite suppressant effect observed when TNP (N2-(m-Trifluorobenzyl), N6-(p-nitrobenzyl)purine) was administered to the mouse ventricle:
   Saline control: saline treated group;
   TNP 0.4 pg: 0.4 pg of TNP treated group;
   TNP 0.04 ng: 0.04 ng of TNP treated group; and
   TNP 0.4 ng: 0.4 ng of TNP treated group.
Figure 2 is a diagram illustrating the effect of inhibiting weight gain observed when TNP (N2-(m-Trifluorobenzyl), N6-(p-nitrobenzyl)purine) was administered to the mouse ventricle:
   Saline control: saline treated group;
   TNP 0.4 pg: 0.4 pg of TNP treated group;
   TNP 0.04 ng: 0.04 ng of TNP treated group; and
   TNP 0.4 ng: 0.4 ng of TNP treated group.

### DESCRIPTION OF THE PREFERRED EMBODIMENTS

Hereinafter, the present invention is described in detail.

The present invention provides a pharmaceutical composition for use in suppressing appetite in a subject in need thereof comprising the TNP (N2-(m-Trifluorobenzyl), N6-(p-nitrobenzyl)purine) compound represented by formula 1 below or a pharmaceutically acceptable salt thereof as an active ingredient:

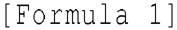

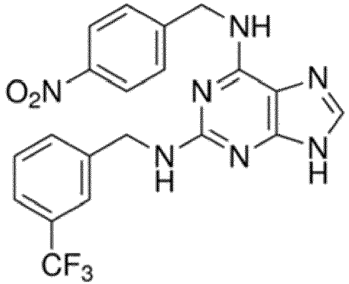

The TNP compound above is characterized by reducing food intake.

In a preferred embodiment of the present invention, TNP compound was administered through the cannula inserted in the third ventricle of the male mouse, and the food intake was measured. As a result, it was confirmed that the food intake of the mouse was reduced (see Figures 1 and 2).

Therefore, the TNP compound of the present invention can be effectively used as a pharmaceutical composition for suppressing appetite.

The pharmaceutical composition of the present invention can additionally include excipients, disintegrants, sweeteners, lubricants, and flavors, etc. The composition of the invention can be formulated as tablets, capsules, powders, granules, suspensions, emulsions, syrups, and other liquid preparations by the conventional methods.

Particularly, the pharmaceutical composition of the present invention can be formulated for oral administration, for example as tablets, troches, lozenges, aqueous or oily suspensions, powders or granules, emulsions, hard or soft capsules, syrups or elixirs. To formulate the composition of the present invention as tablets and capsules, binders such as lactose, saccharose, sorbitol, mannitol, starch, amylopectin, cellulose or gelatin; excipients such as dicalcium phosphate; disintegrants such as corn starch or sweet potato starch; and lubricants such as magnesium stearate, calcium stearate, sodium stearyl fumarate or polyethylene glycol wax can be contained. In the case of capsules, liquid carriers such as fatty oil can be contained in addition to the above-mentioned substances.

The pharmaceutical composition of the present invention can be administered by orally or parenterally and the parenteral administration includes subcutaneous injection, intravenous injection, intramuscular injection and intrathoracic injection. To prepare a formulation for parenteral administration, the TNP compound of the present invention is mixed with a stabilizer or a buffering agent to produce a solution or suspension, which is then formulated as ampoules or vials.

The effective dosage of the pharmaceutical composition of the present invention can be determined according to absorptiveness of the active ingredient, inactivation rate, excretion rate, age, gender, health condition and severity of a disease by those in the art. In the case of oral administration, the pharmaceutical composition can be administered by 0.0001 - 500 mg/kg per day for an adult, and more preferably by 0.001 - 100 mg/kg per day. The administration frequency is once a day or a few times a day.

The pharmaceutical composition of the present invention can be administered alone or treated together with surgical operation, hormone therapy, chemo-therapy and biological regulators.

The present invention also provides a nutraceutical food for use in suppressing appetite in a subject in need thereof comprising the TNP compound represented by formula 1 below or a pharmaceutically acceptable salt thereof as an active ingredient:

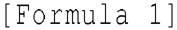

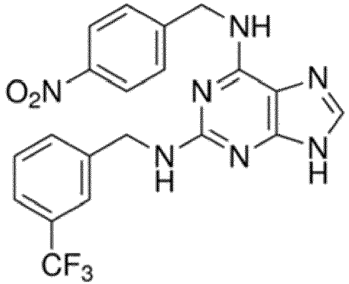

In a preferred embodiment of the present invention, TNP compound was administered through the cannula inserted in the third ventricle of the mouse, and the food intake was measured. As a result, it was confirmed that the food intake of the mouse was reduced (see Figures 1 and 2), and the effect of the TNP compound on the appetite suppression.

The oral administration of the TNP compound of the invention can bring the same effect as the above, so that the TNP compound of the present invention can be effectively used as a nutraceutical food for suppressing appetite.

In this description of the present invention, the term "nutraceutical food" is the food prepared with such a raw material or a component that is likely to be deficient in a daily meal, or has beneficial function for human body (functional raw material), which is defined by Ministry of Food and Drug Safety as the food to maintain or improve health by maintaining the normal function or by activating the physiological function of the human body, but not always limited thereto and does not exclude any conventional health food in its meaning.

The TNP compound of the present invention can be used as a food additive. In that case, the TNP compound of the present invention can be added as it is or as mixed with other food components according to the conventional method.

The nutraceutical food of the present invention can additionally contain sitologically acceptable food additives. The sitologically acceptable food additive that can be used in the present invention includes natural carbohydrates such as sugars such as glucose, fructose, maltose, sucrose, dextrin and cyclodextrin and sugar alcohols such as xilytole, sorbitol and erythritol; natural flavors such as thaumatin and stevia extract; synthetic flavors such as saccharin and aspartame; coloring agents; pectic acid and its salts; alginic acid and its salts; organic acids; protective colloidal viscosifiers; pH adjusting agents; stabilizers; preservatives; glycerin; alcohols; and carbonating agents, but not always limited thereto.

In addition to the ingredients mentioned above, the nutraceutical food of the present invention can include in variety of nutrients, vitamins, minerals (electrolytes), flavors including natural flavors and synthetic flavors, coloring agents and extenders (cheese, chocolate, etc.).

Practical and presently preferred embodiments of the present invention are illustrative as shown in the following Examples.

However, it will be appreciated that those skilled in the art, on consideration of this disclosure, may make modifications and improvements within the scope of the present invention.

### Example 1: Cannulation in the third ventricle of mouse

Adult male C57BL/6 mice were purchased from Orient Bio (Gyeonggi-do, Korea) and the mice were allowed to take the standard diet (Agri Purina, Seoul, Korea) freely. The mice were raised in the controlled condition (temperature: 22 ± 1°C, light/dark cycle: 12/12 hr (light condition: 8:00 am ∼ 8:00 pm)). A cannula was intubated in the third ventricle of the mouse for the administration of TNP. For the cannulation, the mouse was anesthetized with a mixture of Zoletil and Rumpun (2:1 v/v, 10 µℓ/g weight) and then a permanent 26-gauge stainless steel cannula was inserted in the third ventricle of the mouse. The insertion position of the cannula was 1.8 mm back from the bregma and 5.0 mm down from the sagittal sinus. The exact cannulation site was confirmed by the positive dipsogenic response after the administration of angiotensin II (50 ng). Only those mice that were confirmed to have cannulae in the correct location were used for data analysis. After the 7 day recovery period, the mice were massaged every day for 1 week in order to minimize the stress response to the experiment.

### Example 2: Administration of TNP (N2-(m-Trifluorobenzyl), N6-(p-nitrobenzyl)purine)

The mice cannulated in Example 1 were fasted for 18 hours and then administered at different concentrations of TNP (0.4 pg, 0.04 ng, and 0.4 ng; Calbiochem) in the early light phase (9:00 am ∼ 11:00 am). Before the administration, TNP was dissolved in 0.9% saline. 2 µℓ of the TNP dissolved in saline at different concentrations (0.4 pg, 0.04 ng, and 0.4 ng) was administered via the cannula into the third ventricle. For the control, the same amount of saline was administered. After the administration, food intake and body weight were observed for 24 hours.

### Experimental Example 1: Effect of single administration of TNP in the third ventricle on food intake

5 mice treated with saline, 5 mice treated with 0.4 pg of TNP, 5 mice treated with 0.04 ng of TNP, and 4 mice treated with 0.4 ng of TNP were fasted for 18 hours as described in Example 2. TNP was administered once into the third ventricle of the mice. Then, the mice were allowed to take food freely and the food intake was measured for 24 hours.

As a result, as shown in Figure 1, the food intake was significantly reduced in the mice administered with TNP, compared to the mice administered with saline. The food intake reduction effect was significant from 2 hours after the administration and the effect was still maintained after 24 hours after the administration. Particularly, the significance of p<0.1 was confirmed at the time point of 1 hr with 0.4 pg; 2 hr with 0.4 pg and 0.04 ng; 4 hr with 0.4 pg; 4 hr with 0.04 ng; and 24 hr with 0.4 pg, compared to the control treated with saline. All the other experimental groups showed statistical significance of p<0.05.

All experimental values of the present invention were expressed as mean ± standard error (mean ± SD). Statistical analysis was performed by Student's t-test. Significance limits were set at p<0.05 (**) and p<0.1 (*), respectively.

### Experimental Example 2: Effect of single administration of TNP in the third ventricle on weight control

0.4 pg, 0.04 ng, and 0.4 ng of TNP were administered once into the third ventricle of the mice fasted for 18 hours as described in Example 2. Then, the mice were allowed to take food freely. Weight changes of the mice were observed for 24 hours.

As a result, as shown in Figure 2, weight gaining was significantly suppressed for 24 hours in the mice treated with TNP, compared to the control mice treated with saline.

The above results indicate that the administration of TNP in the third ventricle continuously induces a reduction of food intake and inhibits weight gaining.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the scope of the invention as set forth in the appended Claims.

## Claims

1. A pharmaceutical composition comprising the TNP (N2-(m-Trifluorobenzyl), N6-(p-nitrobenzyl)purine) compound represented by formula 1 below, or a pharmaceutically acceptable salt or a pharmaceutical composition thereof, for use in suppressing appetite in a subject in need thereof.

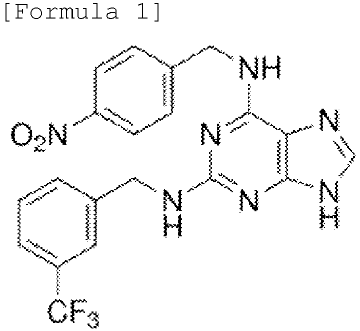

2. A pharmaceutical composition for use according to claim 1, wherein the TNP compound is **characterized by** reducing food intake.

3. A health functional food comprising the TNP (N2-(m-Trifluorobenzyl), N6-(p-nitrobenzyl)purine) compound represented by formula 1 below, or a pharmaceutically acceptable salt thereof as an active ingredient, for use in suppressing appetite in a subject in need thereof.

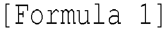

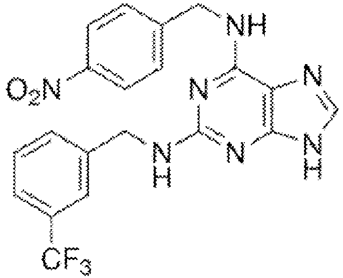

## Patentansprüche

1. Eine pharmazeutische Zusammensetzung, die die Verbindung TNP (N2-(m-Trifluorbenzyl),N6-(p-nitrobenzyl)purin), die durch die nachstehende Formel 1 dargestellt ist, oder ein pharmazeutisch annehmbares Salz oder eine pharmazeutische Zusammensetzung davon umfasst, zur Verwendung zur Appetitunterdrückung bei einem Subjekt, das diese benötigt.

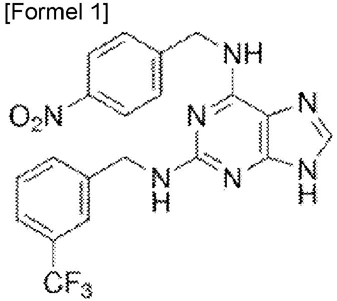

2. Eine pharmazeutische Zusammensetzung zur Verwendung gemäß Anspruch 1, wobei die TNP-Verbindung **gekennzeichnet ist durch** Verringerung der Nahrungsaufnahme.

3. Eine funktionale Reformkost, die die Verbindung TNP (N2-(m-Trifluorbenzyl),N6-(p-nitrobenzyl)purin), die durch die nachstehende Formel 1 dargestellt ist, oder ein pharmazeutisch annehmbares Salz davon als einen Wirkstoff umfasst, zur Verwendung zur Appetitunterdrückung bei einem Subjekt, das diese benötigt.

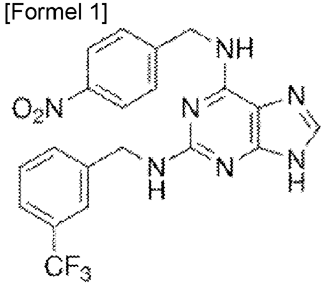

## Revendications

1. Composition pharmaceutique comprenant le composé TNP (N2-(m-trifluorobenzyle),N6-(p-nitrobenzyl)purine) représenté par la formule 1 ci-dessous, ou un sel pharmaceutiquement acceptable ou une composition pharmaceutique de celui-ci, pour une utilisation dans la suppression de l'appétit chez un sujet en ayant besoin.

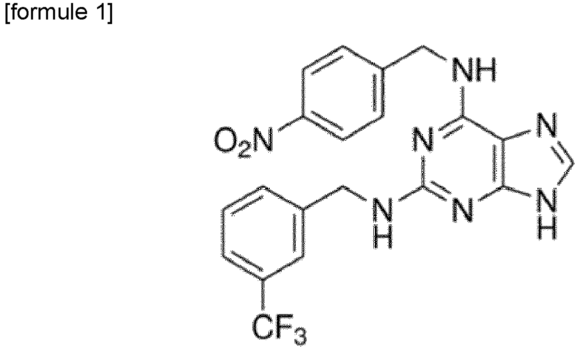

2. Composition pharmaceutique pour une utilisation selon la revendication 1, dans laquelle le composé TNP est **caractérisé en ce qu'**il réduit la prise d'aliments.

3. Aliment fonctionnel de santé comprenant le composé TNP (N2-(m-trifluorobenzyle),N6-(p-nitrobenzyl)purine) représenté par la formule 1 ci-dessous, ou un sel pharmaceutiquement acceptable de celui-ci, en tant que principe actif, pour une utilisation dans la suppression de l'appétit chez un sujet en ayant besoin.
